(19) **Europäisches Patentamt / European Patent Office / Office européen des brevets**

(11) **EP 2 996 712 B1**

(12) **EUROPEAN PATENT SPECIFICATION**

(45) Date of publication and mention
of the grant of the patent:
**31.10.2018 Bulletin 2018/44**

(51) Int Cl.:
*A61K 38/46* (2006.01)     *A61K 38/47* (2006.01)
*A61K 38/48* (2006.01)     *A23L 29/00* (2016.01)
*A23L 33/20* (2016.01)     *A61K 47/06* (2006.01)
*C12N 9/20* (2006.01)

(21) Application number: **14717867.7**

(22) Date of filing: **13.03.2014**

(86) International application number:
**PCT/IB2014/059722**

(87) International publication number:
**WO 2014/141121 (18.09.2014 Gazette 2014/38)**

(54) **PROCESS FOR THE PREPARATION OF A COMPOSITION CONTAINING DIGESTIVE ENZYMES AND NUTRIENTS SUITABLE FOR ENTERAL ADMINISTRATION**

VERFAHREN ZUR HERSTELUNG EINER ZUSAMMENSETZUNG MIT VERDAUUNGSENZYMEN UND NÄHRSTOFFEN ZUR ENTERALEN VERABREICHUNG

PROCÉDÉ DE PRÉPARATION D'UNE COMPOSITION CONTENANT DES ENZYMES DIGESTIVES ET NUTRIMENTS APPROPRIÉS POUR UNE ADMINISTRATION ENTÉRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361798027 P**

(43) Date of publication of application:
**23.03.2016 Bulletin 2016/12**

(73) Proprietor: **Allergan Pharmaceuticals International Limited**
**Dublin 17, D17 E400 (IE)**

(72) Inventors:
• **PIRONTI, Vincenza**
**I-20060 Milan (IT)**
• **RONDA, Emanuela**
**I-20060 Milan (IT)**
• **BOLTRI, Luigi**
**I-20060 Milan (IT)**

(74) Representative: **Kremer, Simon Mark et al**
**Mewburn Ellis LLP**
**City Tower**
**40 Basinghall Street**
**London EC2V 5DE (GB)**

(56) References cited:
**WO-A1-01/70047     WO-A1-2012/019186**
**WO-A2-2008/102264     WO-A2-2012/052853**
**WO-A2-2015/020943**

• **FERRIE S ET AL: "Pancreatic enzyme supplementation for patients receiving enteral feeds", NUTRITION IN CLINICAL PRACTICE 2011 SAGE PUBLICATIONS LTD USA, vol. 26, no. 3, June 2011 (2011-06), pages 349-351, XP009178443, ISSN: 0884-5336**
• **CHEN YIMIN ET AL: "Enteral nutrition formulas: which formula is right for your adult patient?", NUTRITION IN CLINICAL PRACTICE : OFFICIAL PUBLICATION OF THE AMERICAN SOCIETY FOR PARENTERAL AND ENTERAL NUTRITION 2009 JUN-JUL, vol. 24, no. 3, June 2009 (2009-06), pages 344-355, XP009178429, ISSN: 0884-5336**

**Description**

FIELD OF THE INVENTION

The present invention is directed to a process for the preparation of a stable and homogeneous liquid composition, comprising a digestive enzyme product and nutrients from a nutritional formula, that is suitable for enteral administration. The process comprises the preparation of a digestive enzyme product pre-suspension and its addition to the nutritional formula. Also disclosed herein is a method for efficiently and effectively administering a therapeutically effective dose of the stable and homogeneous liquid composition, comprising a digestive enzyme product and nutrients from a nutritional formula by means of an enteral tube.

BACKGROUND OF THE INVENTION

The proper dosing of medications for patients is an important concern within the medical field. For infants, smaller children, and geriatric patients in particular, as well as sometimes also in adult populations, the administration of medications and dosing methods often present substantial issues. As is well known in the art, medications are provided in many forms (e.g., liquid, solid, and combinations of solids in liquids) and are delivered to patients in many ways (e.g., orally, via injection, transdermally). Nevertheless, there is still a need to optimize pancreatic enzyme supplement dosage formulations to improve both their efficacy and patient compliance in their use. Thus, for patients suffering from conditions in which pancreatic enzymes are routinely used (such as exocrine pancreatic insufficiency, EPI) what is in question is how to get a pancreatic enzyme supplement to be the most efficacious at the lowest dose, and have a well-defined safety profile.

In cases of exocrine pancreatic insufficiency (EPI), of which the FDA estimates that more than 200,000 Americans suffer, patients are incapable of properly digesting food due to a lack of digestive enzymes made by their pancreas. That loss of digestive enzymes leads to disorders such as the maldigestion and malabsorption of nutrients, which lead to malnutrition and other consequent undesirable physiological conditions associated therewith. These disorders are common for those suffering from cystic fibrosis (CF) and other conditions compromising the insufficient exocrine function of the pancreas, such as pancreatic cancer, pancreatectomy, and pancreatitis. This malnutrition can be life threatening if left untreated, particularly in the case of infants, and CF patients and the disorders lead to impaired growth in children, compromised immune response, and shortened life expectancy.

Other conditions in which pancreatic enzymes are routinely used are usually conditions that alter the gastrointestinal anatomy (gastric bypass, pancreaticoduodenectomy, small bowel resection, etc.) or impair gut function that results in malabsorption (celiac disease, Crohn's disease, diabetes, bacterial overgrowth, etc.) or other secondary physiological conditions that alter absorption (gastrointestinal tumors, pharmacological agents [i.e. octreotide], etc.).

Digestive enzymes, such as pancrelipase enzymes and other pancreatic enzymes products (PEPs) can be administered to at least partially remedy EPI. The administration of digestive enzyme supplements allows patients to more effectively digest their food.

Pancrelipase enzymes used for treating EPI are mainly a combination of three enzyme classes: lipase, protease and amylase, together with their various co-factors and co-enzymes. These enzymes are produced naturally in the pancreas and are important in the digestion of fats, proteins and carbohydrates. Pancrelipase enzymes are typically prepared from porcine pancreatic glands, although other sources can also be used, for example those described in U.S. 6,051,220, U.S. 2004/0057944, 2001/0046493, and WO2006044529. The enzymes catalyze the hydrolysis of fats into glycerol and fatty acids, starch into dextrin and sugars, and proteins into amino acids and derived substances.

Pancreatic enzymes show optimal activity under near neutral and slightly alkaline conditions. Under gastric conditions, pancreatic enzymes may be inactivated with a resulting loss in biological activity; pancreatic lipases, which are key in the treatment of malabsorption, are especially sensitive to gastric inactivation. Thus, lipase activity is typically monitored to determine the stability of an enzyme composition containing lipase.

Composition containing digestive enzymes, such as pancrelipase enzymes, have been developed for oral administration in form of capsules (Zenpep®, Creon®, Cotazym® and Pancreaze®), tablets (Viokace™, Viokase®), granules (Eurobiol®). However, if a patient is unable to swallow the capsules, each capsule can be opened and the contents sprinkled on a small amount of food, usually a soft, acidic food (such as commercially available applesauce) and administered orally to the patient with a spoon. Alternatively such medications may be administered orally for infants and children, using a syringe device containing the contents suspended in a medium amenable to administration thereby.

It is also recognized that for some patients, including pediatric and adult patients with EPI, feeding through enteral tubes, including smaller lumen enteral feeding tubes, such as gastric and jejunal feeding tubes, is required. Thus, there is a clear need for the enteral administration of digestive enzymes, such as pancrelipase enzymes, to such patients who are unable to take digestive enzymes orally. Where the digestive enzymes are in form of particles, they can be added into a nutritional formula for administration, however issues include how to ensure that the digestive enzymes effectively exert their enzyme activity on constituents susceptible thereto in the nutrients formula and to obviate potential obstructions to enteral feeding by the particulates. Use of tablet forms of digestive enzyme products also suffers for the same reasons.

WO 2012042372 discloses methods for preparing predigested nutritional formula for administration to a patient including by enteral administration. The reference discloses how to mechanically or chemically treat enteric coated pancreatic enzyme products in order to dissolve the coating and liberate the enzyme to be effective for digesting the nutritional formula. The mixture is very complex in term of ingredients and enzymatic reactions which occur during administration to the patients, and that can be unstable, and give rise to the separation of lipid and aqueous phases and precipitation of insoluble components are likely to occur. This reference does not disclose how to prepare a predigested nutritional formula that is sufficiently stable and homogeneous so as to be suitable for enteral administration.

Enteral feeding can be given through: the mouth (orogastric tube or OG); the nose (nasogastric tube or NG); the stomach (gastrostomy or GT); the intestine (jejunostomy or JT); they can be used to deliver calories and nutrients while sleeping at night or during the daytime. A nasogastric feeding tube, or "NG-tube," is passed through the nose, down the esophagus and into the stomach. Gastric feeding tubes, or "G-tube," on the other hand, are inserted through a small incision in the abdomen directly into the stomach, and are increasingly becoming the standard care for many patients, such as cystic fibrosis patients who exhibit chronic weight loss and require long-term enteral nutrition.

Regardless of the route of entry, longer feeding tubes (OG or NG) are also used to deliver nutrients directly to the duodenum or jejunum, bypassing stomach.

Placement of a feeding tube is contingent upon a variety of conditions, including the overall patient health and age, severity of the condition, duration of placement, type of tube, means of placement, patient comfort, mitigating complications, potential for infection, financial considerations, availability, access and use. Thus, a variety of tubes are available in a number of sized for such applications.

Short-term benefits of enteral feeding include immediate weight gain and increased energy. Long term gains include an increase in body fat, lean muscle mass, improved strength, a stronger immune system, less weight loss during pulmonary infections, a greater sense of control over body weight and numerous other benefits.

However, despite the obvious benefits offered by enteral nutrition, gastrostomy administration of solid oral dosage medicines is complicated by a number of preparative and administrative challenges that may render the active pharmaceutical ingredients ineffective. It is also mandatory to have available stable and homogenous complex composition to ensure consistent and complete delivery of the pancrelipase enzymes through the syringe outlet and through the lumen of the G-tube without clogging, or sticking.

Ferrie et al. Nutr. Clin. Pract. 2011, 26(3): 349-51 discloses a technique for administration of pancreatic enzyme via feeding tubes which involves opening the pancreatin capsules and suspending the enzyme microspheres in thickened acidic fluid for delivery into the feeding tube.

WO2012019186 discloses a process for the preparation of a predigested nutritional formula comprising digestive enzymes and a liquid nutritional composition comprising a mixture of carbohydrates, lipids, proteins and water.

WO0170047 discloses a nutritional composition for an infant formula which comprises hydrolysed protein and a method of production of the composition.

In view of the aforesaid, there is a need for a quick, practical, cheap, simple and effective process for preparing a digestive enzymes nutritional composition that can be applied by different people and with different equipments; more particularly to compositions that are stable and homogeneous for a suitable period of time that would be capable of enteral administration without any phase separation and susceptibility to obstruction of an enteral feeding tube.

## SUMMARY OF THE INVENTION

The present invention is directed to a process for the preparation of a stable and homogeneous liquid composition, comprising a digestive enzyme product and nutrients from a specific nutritional formula, that is suitable for enteral administration, as defined in the claims. The process comprises the preparation of a digestive enzyme product presuspension and its addition to the nutritional formula. The disclosure further provides a method for efficiently and effectively administering a therapeutically effective dose of the stable and homogeneous liquid composition, comprising a digestive enzyme product and nutrients from a nutritional formula by means of an enteral tube.

## DETAILED DESCRIPTION OF THE INVENTION

The present invention is directed to a process for the preparation of a stable and homogeneous liquid composition comprising a digestive enzyme product and nutrients from a nutritional formula, said process comprising the preparation of a suspension of digestive enzyme product in aqueous solution followed by mixing said suspension with a liquid nutritional formula containing nutrients in specific amount, as defined in the claims. This liquid composition retains the enzymatic activities (lipase, amylase, protease) for at least about 8 hours from its preparation.

The preparation of a suspension of digestive enzymes comprises the steps of: a.1) reducing the size of the digestive enzymes by means such as crushing, pulverizing or mashing; a.2) adding an aqueous solution; and a.3) mixing the aqueous solution and the digestive enzymes to form the suspension, as defined in the claims. The obtained digestive enzyme suspension is held for a short period of time before it is added to a liquid nutritional formula having specific amount of nutrients.

The digestive enzyme product used according to the invention may be in any suitable dosage forms including tablets, capsules, granules, or sachets. Suitable digestive enzymes product useful according to the invention is preferably a non-gastroresistant pancrelipase enzyme. A non-gastroresistant product is a product which is not intended to resist in gastric fluid. A non-gastroresistant product may be uncoated or coated. If this product is coated, the coating dissolves in gastric fluid. The coating is preferably a pH independent water soluble polymer. The coating may be also a pH dependent water soluble polymer but in this case it is present in such a very small amount and/or if it is non homogenously present on the product thus leaving the product easily and directly exposed to the gastric environment, and therefore non-gastroresistance is observed.

The terms uncoated or coated identified the absence or the presence, respectively, of a polymeric layer around the product. Examples of of pH independent water soluble polymers are: hydroxypropylmethylcellulose, hydroxypropylcellulose, methylcellulose, polyvinylpyrrolidone, or polyvinyl alcohol. Examples of pH-dependent water soluble polymers are: cellulose acetate phthalate, hydroxypropyl methylcellulose phthalate, hydroxypropylmethylcellulose acetate succinate, shellac, methylmethacrylate copolymers, and methacrylic acid/ methylmethacrylate copolymers, methacrylic acid-ethyl acrylate copolymer (1:1) (such as Eudragit® L30D55). The pancrelipase enzyme product for use according to the invention is preferably uncoated.

The digestive enzyme product useful for the present invention may be any immediate release pancrelipase enzyme product or dosage form.

Examples of such pancrelipase enzyme products include Viokace™ (marketed in USA), Viokase® (marketed in Canada), Eurobiol® 12,500 PhEur lipase units (marketed in France) and Cotazym® (marketed in Canada).

The term "digestive enzyme" used herein denotes an enzyme in the alimentary tract which breaks down the components of food so that they can be taken or absorbed by the organism. Non-limiting examples of digestive enzymes include pancrelipase enzymes (also referred to as pancrelipase enzymes or pancreatin), lipase, co-lipase, trypsin, chymotrypsin, chymotrypsin B, pancreatopeptidase, carboxypeptidase A, carboxypeptidase B, glycerol ester hydrolase, phospholipase, sterol ester hydrolase, elastase, kininogenase, ribonuclease, deoxyribonuclease, $\alpha$-amylase, papain, chymopapain, glutenase, bromelain, ficin, $\beta$-amylase, cellulase, $\beta$-galactosidase, lactase, sucrase, isomaltase, and mixtures thereof.

The digestive enzymes include powder, granules, tablets, spheres, minitablets, microtablets, microparticles, microspheres, microcapsules, micropellets, as well as any particles having diameters up to about 5 mm; the particle may have any size or shape.

The term "pancreatic enzyme" as used herein refers to any one of the enzyme types present in the pancreatic secretion, such as amylase, lipase, protease, or mixtures thereof, or any extractive of pancreatic origin having enzymatic activity, such as pancreatin.

The terms "pancrelipase enzymes" or "pancrelipase enzymes" or "pancreatin" denotes a mixture of several types of enzymes, including amylase, lipase, and protease enzymes. Pancrelipase enzyme is commercially available, for example from Nordmark Arzneimittel GmbH, or Scientific Protein Laboratories LLC.

The term "lipase" denotes an enzyme that catalyzes the hydrolysis of lipids to glycerol and simple fatty acids. Examples of lipases suitable for the present invention include, but are not limited to animal lipase (e.g., porcine lipase), bacterial lipase (e.g., Pseudomonas lipase and/or Burkholderia lipase), fungal lipase, plant lipase, recombinant lipase (e.g., produced via recombinant DNA technology by a suitable host cell, selected from any one of bacteria, yeast, fungi, plant, insect or mammalian host cells in culture, or recombinant lipases which include an amino acid sequence that is homologous or substantially identical to a naturally occurring sequence, lipases encoded by a nucleic acid that is homologous or substantially identical to a naturally occurring lipase-encoding nucleic acid, etc.), synthetic lipase, chemically-modified lipase, and mixtures thereof. The term "lipids" broadly includes naturally occurring molecules including fats, waxes, sterols, fat- soluble vitamins (such as vitamins A, D, E and K), monoglycerides, diglycerides, triglycerides, phospholipids, etc.

The term "amylase" refers to glycoside hydrolase enzymes that break down starch, for example $\alpha$-amylases, $\beta$-amylases, $\gamma$-amylases, acid $\alpha$-glucosidases, salivary amylases such as ptyalin, etc. amylases suitable for use in the present invention include, but are not limited to animal amylases, bacterial amylases, fungal amylases (e.g., Aspergillus amylase, for example, Aspergillus oryzae amylase), plant amylases, recombinant amylases (e.g., produced via recombinant DNA technology by a suitable host cell, selected from any one of bacteria, yeast, fungi, plant, insect or mammalian host cells in culture, or recombinant amylases which include an amino acid sequence that is homologous or substantially identical to a naturally occurring sequence, amylases encoded by a nucleic acid that is homologous or substantially identical to a naturally occurring amylase-encoding nucleic acid, etc.), chemically modified amylases, and mixtures thereof.

The term "protease" refers generally to enzymes (e.g., proteinases, peptidases, or proteolytic enzymes) that break peptide bonds between amino acids of proteins. proteases are generally identified by their catalytic type, e.g., aspartic acid peptidases, cysteine (thiol) peptidases, metallopeptidases, serine peptidases, threonine peptidases, alkaline or semi- alkaline proteases, neutral and peptidases of unknown catalytic mechanism. Non-limiting examples of proteases suitable for use in the present invention include serine proteases, threonine proteases, cysteine proteases, aspartic acid proteases (e.g., plasmepsin), metalloproteases and glutamic acid proteases. In addition, proteases suitable for use in the present invention include, but are not limited to animal proteases, bacterial proteases, fungal proteases (e.g., an Aspergillus melleus protease), plant proteases, recombinant proteases (e.g., produced via recombinant DNA technology by a suitable host cell, selected from any one of bacteria, yeast, fungi, plant, insect or mammalian host cells in culture, or recombinant proteases, which include an amino acid sequence that is homologous or substantially identical to a naturally occurring sequence, proteases encoded by a nucleic acid that is homologous or substantially identical to a naturally occurring protease-encoding nucleic acid, etc.), chemically modified proteases, and mixtures thereof.

The pancrelipase enzymes of the composition of present invention can include one or more lipases (i.e., one lipase, or two or more lipases), one or more amylases (i.e., one amylase, or two or more amylases), one or more proteases (i.e., one protease, or two or more proteases), as well as mixtures of these enzymes in different combinations and ratios.

Lipase activities in the compositions useful for the present invention can be from about 650 to about 45,000 IU (USP method) (or 45,000 USP units), from about 675 to about 825 IU, from about 2,500 to about 28,000 IU, from about 2,700 to about 3,300 IU, from about 4,500 to about 5,500 IU, from about 8,000 to about 11,000 IU, from about 13,500 to about 16,500 IU, and from about 18,000 to about 22,000 IU, from about 22,500 to about 27,500 IU, from about 36,000 to about 44,000 IU, and all ranges and subranges there between. Also the lipase activity can range from about 5,000 PhEur lipase units to about 30,000 PhEur lipase units, it may be about 5,000, or about 10,000, or about 12,500, about 15,000 or about 20,000 or about 30,000, or about 40,000 PhEur lipase units.

Amylase activities in the compositions can be from about 1,600 to about 6,575 IU (USP) (or 6,575 USP units), from about 6,000 to about 225,000 IU, for example from about 6,400 to about 26,300 IU, from about 10,700 to about 43,800 IU, from about 21,500 to about 87,500 IU, from about 32,100 to about 131,300 IU, from about 42,900 to about 175,000 IU, from about 53,600 to about 218,700 IU and all ranges and subranges there between.

Protease activities in the compositions can be from about 1,250 to about 3,850 IU (USP) (or 3,850 USP units), from about 5,000 to about 130,000 IU, for example from about 5,000 to about 15,400 IU, from about 8,400 to about 25,700 IU, from about 16,800 to about 51,300 IU, from about 25,000 to about 77,000 IU, from about 33,500 to about 102,800 IU, from about 41,800 IU to about 128,300 IU and all ranges and subranges there between.

The lipase activity can range from about 675 to about 825 IU (or 825 USP units), the amylase activity from about 1,600 to about 6,575 IU, and the protease activity from about 1,250 to about 3,850 IU (USP). Or the lipase activity can range from about 2,700 to about 3,300 IU, the amylase activity from about 6,400 to about 26,300 IU, and the protease activity from about 5,000 to about 15,400 IU (USP) (or 15,400 USP units). Or the lipase activity can range from about 4,500 to about 5,500 IU, the amylase activity from about 10,700 to about 43,800 IU, and the protease activity from about 8,400 to about 25,700 IU (USP) (or 25,700 USP units). Or the lipase activity can range from about 9,000 to about 11,000 IU, the amylase activity from about 21,500 to about 87,500 IU, and the protease activity from about 16,800 to about 51,300 IU (USP) (or 51,300 USP units). Or the lipase activity from about 13,500 to about 16,500 IU, the amylase activity from about 32,100 to about 131,300 IU, and the protease activity from about 25,000 to about 77,000 IU (USP) (or 77,000 USP units). The lipase activity can range from about 18,000 to about 22,000 IU, the amylase activity from about 42,900 to about 175,000 IU, and the protease activity from about 33,500 to about 102,600 IU (USP) (or 102,500 USP units). The lipase activity can range from about 22,000 to about 27,500 IU, the amylase activity from about 53,600 to about 218,700 IU, and the protease activity from about 41,800 IU to about 128,300 IU (USP) (or 128,300 USP units).

In one embodiment of the present invention also single units containing a fraction of the above listed amylase activities can be used in the present process. In the process of the invention an effective amount of pancrelipase enzymes used to prepare the suspension; said effective amount of enzymes may be of a total of about 3,000, about 4,200, about 5,000, about 6,000, about 8000, about 10,000, about 10,440, about 10,500, about 15,000, about 16,000, about 16,800, 16,800, about 20,000, about 20,880, about 21,000, about 24,000, or 25,000 USP, lipase units or multiple thereof, or about 5,000, or about 12,500, or about 30,000 PhEur lipase units or multiple thereof.

In the invention the preparation of the digestive enzyme suspension comprises the steps of: a.1) reducing the size of the digestive enzymes (preferably non-gastroresistant) by means such as crushing, pulverizing or mashing; a.2) adding an aqueous solution; and a.3) mixing to form the suspension, as defined in the claims. The obtained suspension is held for a short period of time before it is added to the liquid nutritional formula having specific amount of nutrients; this period of time should be greater than five minutes, more preferably greater than 10 minutes; it is preferably comprised from about 15 to about 30 minutes; preferably it is held for about 15 minutes.

In the first step a.1) the pancrelipase enzymes particles are crushed to obtain a fine powder; no dose loss should occur during this step which may be performed either with a manual process (using: ceramic mortar and pestle; coffee mug and metal spoon) or with a pills crushing device. Crushing devices may be screw (S) types, such as GIMA® (S), Genius® (S), Apex Ultra Pills Crusher® (S). It is preferred to use the pill crusher because of reproducibility both in terms of particles dimension and dose recovery.

The pulverized pancrelipase enzymes are then added to a small volume of suitable administration vehicle, which upon mixing allows the formation of an homogeneous suspension. The administration vehicle is an aqueous solution, which is 1) purified or deionized water, which is comparable to sterile water for non-parenteral administration, except for not complying with sterility requirement; 2) sterile water; 3) physiological solution (0.9% NaCl); or 4) tap water. Purified or sterile water or physiological solution (or saline) is preferred because they are preferred diluents for most drug products.

To obtain an homogeneous digestive enzymes suspension is important to apply a small volume of solution (step a.2) in order to prepare a concentrated suspension with high density; the volume is less than 10 mL. Preferably, the digestive enzymes should be suspended in small volume aqueous solution according to the corresponding strength. In fact, for a dosage of about 9,190 or of about 10,400 USP units of lipase preferably a volume of 2.5 mL is applied; for dosage form having multiple USP units the corresponding multiple volume is applied. As example, one tablet of pancrelipase enzymes with 10,440 USP units of lipase (such as Viokace™) is suspended in 2.5 mL (½ teaspoon) of purified water; one dose of pancrelipase enzymes with 12,500 PhEur units of lipase (such as Eurobiol®, 12,500 PhEur units lipase corresponds to 9,191 USP units; conversion factor from PhEur lipase units to USP lipase units applied is: 1 PhEur units= 1.36 USP units) is suspended in 2.5 mL (½ teaspoon) of purified water; one dose of pancrelipase enzymes with 20,880 USP units of lipase (such as Viokace™) is suspended in 5 mL (1 teaspoon) of purified water. Enzymes stability should be maintained and to attain this a high concentrated suspension should be prepared, dilution factor is a critical aspect for stability because it is directly related to the enzymatic activity degradation.

To obtain an homogeneous suspension is important mixing and then to keep the mixture for a short period of time at room temperature before it is added to a liquid nutritional formula; this period of time should be greater than five minutes, more preferably greater than 10 minutes; it is preferably comprised from about 15 minutes to about 30 minutes. About 15 minutes is particularly suitable for preparing a suspension free of intact particles or fragments of appreciable size independently from the type of crushing or type of pills crusher chosen. This duration of time ensure not only the preparation of an homogeneous suspension but also the lipase activity maintenance. It is in fact important to have always complete dose recovery independent from the pancrelipase enzymes dose strength used. The suspension may be gently stirred with a spoon or spatula for a few seconds before adding it to the nutritional formula.

The pulverized pancrelipase enzyme suspended into the aqueous solvent is then added to the liquid nutritional formula comprising specific amount of nutrients, which are mixture of carbohydrates, lipids, proteins, and water, and then shaken for a suitable period of time, such as for about 15 second before dispensing the composition to the patient from the feeding bag through an enteral tube. The addition of the suspension to the nutritional formula is preferably done directly in the feeding (or dispensing) bag already containing the nutritional formula.

The process of the invention allow the preservation of the given dose strength, thus allowing the complete delivery of the digestive enzyme product; in fact, no loss of enzymatic activity occurs: there is neither degradation nor any mechanical removal of active enzymes during the preparation of the liquid composition.

The present disclosure is also directed to a liquid composition of pancrelipase enzymes and nutrients (Pan+NF) which is a stable and homogeneous dispersion of pancrelipase enzymes in the nutritional formula having specific amount of nutrients. This liquid composition remains stable with regards to the enzymatic activity (lipase, protease and amylase activity). In fact, this mixture retains the lipase activity which is calculated as percentage of the ratio of the lipase activity in the composition at given time (t) to the lipase activity in the nutritional formula at time zero, that is the activity measured immediately after addition of the enzymes to the nutritional formula. After about 8 hours lipase activity is above about 90% or about 100%, protease activity is above about 90% or about 100%, and amylase activity is above 85%, or about 100% at room temperature. The enzymes activity recovery in the digestive enzyme-nutrients composition is the ratio between the enzymatic activity at a given time (t) and that calculated in the mixture immediately after addition, that is at time zero. Moreover, for at least the same period of time of at least about 8-10 hours no phase separation (such as separation between the lipidic and aqueous components, protein precipitation) in the composition is observed. The composition of the disclosure hence allows a constant dose and homogenous nutrients delivery.

This pancrelipase enzymes and nutrients composition is used for nutritional management of impaired gastrointestinal function in pediatric and adults patients and is suitable to be administered via continuous infusion using feeding pump and G-tube without markedly evident phase separation for the administration period.

The nutritional formula used in the present invention may be an adult/children or an infant nutritional formula that comprises specific amount of nutrients, which are mixture of carbohydrates, lipids, proteins; polymeric components that may be in hydrolyzed form. The nutritional formula may further comprises other ingredients such as trace elements and fibers.

The formula useful according to the invention is a nutritional formula having specific amount of nutrients. The total fat and protein and carbohydrate nutrient content is from 10 to 35 g /100 mL; more particularly from about 12 to about 32. When the nutritional formula is an adult/ children nutritional formula the total fat and protein and carbohydrate content is from about 20 to about 32 g /100 mL; and when the nutritional formula is an infant nutritional formula the total fat and protein and carbohydrate nutrient content is from about 12 to about 14 g /100 mL.

Another embodiment useful according to the invention is a nutritional formula having a total fat and protein nutrient content from 4.5 to 11.5 g /100 mL; more particularly from about 4.9 to about 11.3. When the nutritional formula is an adult/ children nutritional formula the total fat and protein content is from about 6.8 to about 11.3 g /100 mL; and when the nutritional formula is an infant nutritional formula the total fat and protein content is from about 4.9 to about 5.3 g /100 mL.

Another embodiment useful according to the invention is a nutritional formula having a total fat nutrient content from 3.0 to 7.0 g /100 mL; more particularly from about 3.3 to about 6.8. When the nutritional formula is an adult/children nutritional formula the total fat content is from about 3.8 to about 6.8 g /100 mL; and when the nutritional formula is an infant nutritional formula the total fat content is from about 3.3 to about 3.7 g /100 mL.

Another embodiment useful according to the invention is a nutritional formula having a total protein nutrient content from 1.3 to 6.3 g /100 mL; more particularly from about 1.4 to about 6.2. Where the nutritional formula is an adult/children nutritional formula the total protein content is from about 3 to about 6.2 g /100 mL; and when the nutritional formula is an infant nutritional formula the total protein content is from about 1.4 to about 1.6 g /100 mL.

Enteral liquid formulas commonly used include polymeric or other specialized formulas. Polymeric formulas including milk-based or lactose-free commercial formulas are commercially available and generally provide a complete, balanced diet. Specialized formulas include hydrolyzed protein or sometimes amino acid formulas, which are used for patients who have difficulty digesting complex proteins.

Commercial liquid adult/ children enteral formula suitable for the present invention are used, such as, but not limited to Peptamen® Junior 1, Peptamen® Junior 1.5, Ensure® Plus, Fortimel® and other similar products may also be used. Example of commercial infant formulas are Humana® 1, Neolatte® 1, and Neolatte® 2.

The digestive enzyme product used in the present invention is a therapeutically effective amount. The pancrelipase enzymes should be dosed into the liquid nutritional formula; the dose may be adapted for individual patients based on the age, clinical symptoms. In the process according to the invention a dose approximately between about 1,000 and about 5,000, preferably between about 1,000 and about 4,500 lipase USP units per g fat in the nutritional formula is recommended as the starting dose when mixed with liquid nutritional formula.

During infusion a modification of the nutrients occurs due to the enzymatic activity of the pancrelipase enzymes on lipids, proteins, carbohydrates, and digested nutrients are formed; this ensures that the digestion occurs. Notwithstanding this change in types and ratios of nutrients and digested products the composition of the invention remains homogenous and stable over 8-10 hours.

A particular embodiment of the invention is a process for the preparation of a stable and homogeneous liquid composition that is suitable for enteral administration comprising a non-gastroresistant pancrelipase enzyme product and nutrients from a nutritional formula, said process comprising the following steps: a) preparing a suspension of pancrelipase enzymes in aqueous solution comprising the step of: a.1) reducing the size of the digestive enzyme product, a.2) adding an aqueous solution in amount of about 2.5 mL for a digestive enzyme product having about 10,400 USP units of lipase, or adding a corresponding multiple amount of solution for a product having multiple USP units of lipase; a.3) mixing to form the suspension; and a.4) keeping it for a period of time greater than about 5 minutes (preferably between about 5 minutes and about 30 minutes; preferably about 15 minutes); and b) mixing the suspension with a liquid nutritional formula to form the stable and homogeneous liquid composition; wherein the nutritional formula has a total fat and protein and carbohydrate nutrient content from 10 to 35 g /100 mL, even more preferably from about 20 to about 32 g /100 mL; and wherein said composition remains stable (no phase separation occurs) for at least eight hours from its preparation and the enzymes have a lipase activity of above about 90% after about 8 hours storage at about room temperature, calculated as percentage of the units of lipase activity added to the liquid nutritional formula.

Another particular embodiment of the invention is a process for the preparation of a stable and homogeneous liquid composition that is suitable for enteral administration comprising a non-gastroresistant pancrelipase enzyme product and nutrients from a nutritional formula, said process comprising the following steps: a) preparing a suspension of pancrelipase enzymes in aqueous solution comprising the step of: a.1) reducing the size of the digestive enzyme product; a.2) adding an aqueous solution in amount of about 2.5 mL for a digestive enzyme product having about 10,400 USP units of lipase, or adding a corresponding multiple amount of solution for a product having multiple USP units of lipase; a.3) mixing to form the suspension; and a.4) keeping it for a period of time greater than about 5 minutes (preferably between about 5 minutes and about 30 minutes; preferably about 15 minutes); and b) mixing the suspension with a liquid nutritional formula to form the stable and homogeneous liquid composition; wherein the nutritional formula has a total fat and protein nutrient content from 4.5 to 11.5 g /100 mL, even more preferably form about 6.8 to about 11.3 g/100 mL; and wherein said liquid composition remains stable (no phase separation occurs) for at least eight hours from its preparation and the enzymes have a lipase activity of above about 90% after about 8 hours storage at about room temperature, calculated as percentage of the units of lipase activity added to the liquid nutritional formula.

Another particular embodiment of the invention is a process for the preparation of a stable and homogeneous liquid composition that is suitable for enteral administration comprising a non-gastroresistant pancrelipase enzyme product and nutrients from a nutritional formula, said process comprising the following steps: a) preparing a suspension of pancrelipase enzymes in aqueous solution comprising the step of: a.1) reducing the size of the digestive enzyme product; a.2) adding an aqueous solution in amount of about 2.5 mL for a digestive enzyme product having about 10,400 USP units of lipase, or adding a corresponding multiple amount of solution for a product having multiple USP units of lipase; a.3) mixing to form the suspension; and a.4) keeping it for a period of time greater than 5 minutes (preferably between about 5 minutes and about 30 minutes; preferably about 15 minutes); and b) mixing the suspension with a liquid nutritional formula to form the stable and homogeneous liquid composition; wherein the nutritional formula has a total fat nutrient content from 3.0 to 7.0 g /100 mL, even more preferably form about 3.8 to about 6.8 g/100 mL; and wherein said liquid composition remains stable (no phase separation occurs) for at least eight hours from its preparation and the enzymes have a lipase activity of above about 90% after about 8 hours storage at about room temperature, calculated as percentage of the units of lipase activity added to the liquid nutritional formula.

Another particular embodiment of the invention is a process for the preparation of a stable and homogeneous liquid composition that is suitable for enteral administration comprising a non-gastroresistant pancrelipase enzyme product and nutrients from a nutritional formula, said process comprising the following steps: a) preparing a suspension of pancrelipase enzymes in aqueous solution comprising the step of: a.1) reducing the size of the digestive enzyme product; a.2) adding an aqueous solution in amount of about 2.5 mL for a digestive enzyme product having about 10,400 USP units of lipase, or adding a corresponding multiple amount of solution for a product having multiple USP units of lipase; a.3) mixing to form the suspension; and a.4) keeping it for a period of time greater than 5 minutes (preferably between about 5 minutes and about 30 minutes; preferably about 15 minutes); and b) mixing the suspension with a liquid nutritional formula to form the stable and homogeneous liquid composition; wherein the nutritional formula has a total protein nutrient content from 1.3 to 6.3 g /100 mL, even more preferably form about 3 to about 6.2 g/100 mL; and wherein said liquid composition remains stable (no phase separation occurs) for at least eight hours from its preparation and the enzymes have a lipase activity of above about 90% after about 8 hours storage at about room temperature, calculated as percentage of the units of lipase activity added to the liquid nutritional formula.

The pancrelipase enzymes and nutrients composition is suitable for administration to infants, children, adults, aged patients, or other patients suffering from EPI, which allows medication to be dispensed carefully and with controlled dosing.

The present disclosure also encompasses a method of administration to pediatric or adult patients of the composition of the digestive enzymes (pancrelipase enzymes) and nutrients of the present disclosure. It comprises the following steps: a) reducing the size of digestive enzymes by means such as crushing, pulverizing or mashing; b) adding small volume of aqueous solution; c) mixing to form the suspension and keeping it for more than 5 minutes; d) mixing the suspension with a liquid nutritional formula having specific amount of nutrients to form the digestive enzyme- nutrients composition either in the dispensing bag or in another container; e) dispensing the composition from the feeding bag to the patient through an enteral tube; the enzymes and nutrients composition may be gently agitated before its dispensing.

The present disclosure describes a reliable procedure suitable for the administration of a liquid pancrelipase enzymes and nutrients composition through gastrostomy-tubes or nasogastric-tubes and ensure consistent delivery of the dose through the lumen of the tube without clogging, sticking and preserving the tube patency. The administration is conducted through different enteral tubes which are chosen according to patients, from newborns, to pediatric, to adult patients. The successful testing of the diameter sizes shown herein indicates that the use of any larger diameter tube of the same type and manufacturer is acceptable when using the described administration procedure.

From the foregoing description and the experimental part, it can be seen that the present invention provides several important advantages. The invention provides a simple and fast process for preparation of a mixture of pancrelipase enzymes and nutrients from a specific nutritional formula; it that remains homogenous for at least about eight hours; the lipase activity is maintained after addition of suspended pancrelipase enzymes into the liquid nutritional formula; the lipase remains stable in the composition for over about eight hours and the lipolysis is effectively achieved.

EXPERIMENTAL

MATERIALS

- Pancrelipase enzymes: pancrelipase enzymes reference standard USP batch 8 (amylase activity assay: 344 USP units/mg, protease activity assay 358 USP units/mg), pancrelipase enzymes reference standard USP batch 3 (lipase activity assay: 93.3 USP units/mg).

- Pancrelipase enzyme product: Viokace™ (10,440 or 20,880 USP units lipase) product marketed in USA; excipients: croscarmellose sodium, colloidal silicon dioxide, cellulose microcrystalline, stearic acid, talc; Viokase® (8,000 or 16,000 USP units lipase) product marketed in Canada; excipients: croscarmellose sodium, colloidal silicon dioxide, cellulose microcrystalline, stearic acid, talc; Eurobiol® 12,500 PhEur units lipase /dose (20 gr) marketed by Mayolyl Spindler; excipients: cellulose microcristalline, crospovidone, colloidal silica anhydrous, magnesium stearate, coating: methacrylic acid- ethyl acrylate copolymer (1:1), trietylcitrate, talc, simethicone emulsion.

- Enteral formula: Peptamen® Junior 1 Cal (Nestle, package of 250 mL, Vanilla, artificial flavor), Peptamen® Junior 1.5 Cal (Nestle, package of 250 mL, unflavored), Ensure® Plus (Abbott Italia package of 200 mL, strawberry artificial flavor), Nutren® 2.0 (Nestle, package of 250 mL), TwoCal® HN (Abbott Nutrition, package of 237 mL), and Fortimel® (Nutricia, package of 200 ml).

- Infant formula: Neolatte®1 (Unifarm, formula is reconstituted as described by manufacturer), Neolatte® 2 (Unifarm, formula is reconstituted as described by manufacturer), Humana® 1 (Unifarm, package of 470 mL), and Nutramigen™ DHA & ARA (Enfamil, package of 946 mL).

The nutrients (fat + protein + carbohydrate) content of enteral and infant formulas is shown in Table 1.

Table 1

| Nutritional Formula | Caloric density (cal/mL) | Fat content g/100 ml | Protein content g/ 100 ml | Carbohydrate content g/100 ml | Fat+ Protein+ Carbohydrate content g/100 mL |
|---|---|---|---|---|---|
| EF1 Peptamen Jr® | 1 | 3.8 | 3.0 | 13.6 | 20.4 |
| EF2 Peptamen® Jr® 1.5 | 1.5 | 6.8 | 4.5 | 18.0 | 29.3 |
| EF3 Ensure® Plus | 1.5 | 4.9 | 6.2 | 20.2 | 313 |
| EF4 Nutren® 2.0 | 2 | 10.4 | 19.6 | 8 | 38 |
| EF5 TwoCal® HN | 2 | 9.0 | 8.4 | 21.8 | 39.2 |
| IF1 Neolatte® 1 | 0.68 | 3.7 | 1.4 | 7.7 | 12.8 |
| IF2 Neolatte® 2 | 0.68 | 3.3 | 1.6 | 8.2 | 13.1 |
| IF3 Humana® 1 | 0.68 | 3.7 | 1.6 | 6.9 | 12.2 |
| IF4 Nutramigen® | 0.68 | 2.1 | 1.1 | 4.1 | 7.3 |

METHODS

*Lipolytic activity*

Measurement is carried out with a method based on the compendia procedure of lipase assay described in the pancrelipase enzymes USP monograph, which is based on the titration, by means of pH-stat method, of the free fatty acids formed from the hydrolysis of esterified fatty acids in the substrate used (olive oil). It is based on the following principle: lipase catalyses the hydrolysis of the triglycerides which leads to the formation of free fatty acids (FFA). The titration of the formed FFA according to time provides for the determination of the enzymatic activity of lipase, which can be expressed in units: 1 U = 1 $\mu$mole of formed FFA per minute. The reaction occurs by maintaining a steady pH value through an experimental system that provides for the addition of NaOH (titrant) when the pH value changes compared to a fixed value (pHstat method). The quantity of added titrant according to time corresponds to the quantity of FFA formed by the lipase action on the triglycerides. The curve slope {added titrant = f (volume (mL)/ time (minutes))} gives the lipase enzymatic activity.

Proteolytic and amilolytic activity measurements are carried out according to the compendial procedure described in the pancrelipase enzymes USP monograph.

*Triglycerides* are extracted with hexane:isopropanol (3:2) using cholesterylpalmitate as internal standard and analyzed by HPLC; peaks are identified by comparing all the retention times with a standard triolein solution.

*Fatty acids* are extracted in hexane: isopropanol (3:2) using stearyl alcohol as internal standard and analyzed by HPLC; peaks are identified by comparing the retention times with fatty acids standards i.e. linoleic acid, palmitic acid, oleic acid and stearic acid.

*Protein analysis* 1) Total Protein Content is quantified with a Bradford Assay; 2) Tryptophan is analysed by HPLC using 5-methyl tryptophan as internal standard.

*Carbohydrate analysis* 1) Short chain sugars are analyzed by HPLC using xylitol as internal standard; peaks are identified by comparing all the retention times with sugars standards i.e. sucrose, maltose and glucose. 2) Maltodextrins is extracted in presence of Carrez I and Carrez II and analyzed by HPLC; peaks are identified by comparing all the retention times with maltodextrins standards i.e. maltose monohydrate, maltotriose, maltotetraose, maltopentaose, maltohexaose and maltoheptaose.

INSTRUMENTS

*Standard equipment for infusion administration* (same as the one used in clinical environment, or at home, mimicking usual feeding procedure) comprises: feeding bag (Kangaroo Joey™ Enteral Feeding Pump Sets), pump (Kangaroo™ ePump Enteral Feeding Pump) and G-tube (Kimberly Clark MIC-KEY, stoma length: 4.0 cm, outer diameter: 12 Fr (12 Fr= 0.33 mm). For the 10 mL/h infusion feeding rate the following parameters are set up: flow: 10 mL/h, flushing with 30 mL of water every 4 four hours. For the 125 mL/h infusion feeding rate the following parameters are set up: flow: 125 mL/h, flushing with 30 mL of water every 4 four hours.

*Pills crushing:* a) manual process, using: ceramic mortar and pestle; coffee mug and metal spoon; b) pills crusher, screw (S) type: GIMA (S), Genius (S), Apex Ultra Pills Crusher® (S).

EXAMPLES

EXAMPLE 1. Suspension of pancrelipase enzymes tablet in administration vehicles

The direct solubilization test of pancrelipase enzymes (Viokase® tablet with 8,000 and 16,000 USP units lipase) in physiological solution and enteral formula EF3 (Ensure® Plus) and EF6 (Fortimel®) is performed by: 1) mixing in the beaker (simulating a cup) with a spatula (mimicking the home spoon) or 2) manually shaken in a bottle (to simulate the original enteral formula packaging). In a fixed small volume of administration vehicle, the tablets (see corresponding dose strength in Table 2) are manually stirred or shaken for 2 minutes and maintained at room temperature. The aspect of the tablets is visually tested (see Table 2). Pancrelipase enzyme tablet provides after 30 minutes a turbid suspension when using physiological solution, whereas the tablet remains intact in enteral formulas EF3 and EF6; 6 hours are required to obtain a suspension in EF3. Both aqueous solution and enteral formulas are not suitable as they do not allow a direct rapid disintegration and solubilization of pancrelipase enzymes; precipitation and phase separation is observed; stable and homogeneous composition cannot be prepared.

Table 2

| Test | Administration vehicle | Dose strength (USP units/tab) | Vol (mL) | Container | Time (min) | Visual aspect of the mixture |
|---|---|---|---|---|---|---|
| 1 | EF3 | 16,000 | 50 | beaker | 5 | unchanged tablet |
| 2 | physiological solution | 16,000 | 50 | beaker | 5 | unchanged tablet |
| 3 | EF6 | 16,000 | 50 | beaker | 10 | unchanged tablet |
| 4 | EF3 | 16,000 | 50 | beaker | 20 | unchanged tablet |
| 5 | EF6 | 16'000 | 50 | beaker | 20 | unchanged tablet |

(continued)

| Test | Administration vehicle | Dose strength (USP units/tab) | Vol (mL) | Container | Time (min) | Visual aspect of the mixture |
|---|---|---|---|---|---|---|
| 6 | physiological solution | 16,000 | 50 | beaker | 20 | turbid suspension |
| 7 | EF3 | 16,000 | 50 | beaker | 30 | unchanged tablet |
| 8 | EF6 | 16,000 | 50 | beaker | 30 | unchanged tablet |
| 9 | physiological solution | 16,000 | 50 | beaker | 30 | turbid suspension |
| 10 | physiological solution | 16,000 | 50 | bottle | 5 | thinning of the tablet |
| 11 | physiological solution | 16,000 | 50 | bottle | 10 | thinning of the tablet |
| 12 | physiological solution | 16,000 | 50 | bottle | 20 | thinning of the tablet |
| 13 | physiological solution | 16,000 | 50 | bottle | 30 | thinning of the tablet |
| 14 | physiological solution | 16,000 | 50 | beaker | 10 | thinning of the tablet |
| 15 | physiological solution | 16,000 | 50 | beaker | 20 | thinning of the tablet |
| 16 | physiological solution | 16,000 | 50 | beaker | 30 | suspension |
| 17 | physiological solution | 16,000 | 50 | bottle | 10 | thinning of the tablet |
| 18 | physiological solution | 16,000 | 50 | bottle | 20 | thinning of the tablet |
| 19 | physiological solution | 16,000 | 50 | bottle | 30 | thinning of the tablet |
| 20 | EF3 | 16,000 | 50 | beaker | 60 | thinning of the tablet |
| 21 | EF3 | 16,000 | 50 | beaker | 360 | Suspension |
| 22 | physiological solution | 16,000 | 50 | beaker | 30 | Suspension |
| 23 | EF3 | 8,000x2 | 50 | beaker | 30 | thinning of the tablet |
| 24 | physiological solution | 8,000x2 | 50 | beaker | 30 | thinning of the tablet |
| 25 | EF3 | 8,000x3 | 200 | beaker | 30 | thinning of the tablet |
| 26 | EF3 | 16,000/2 | 50 | beaker | 30 | thinning of the tablet |

EXAMPLE 2. Tablet crushing

Pancrelipase enzymes tablet (Viokace™) is pulverized using different crushing devices to evaluate the reproducibility of tablet pulverization, dose recovery (without loss), visual aspect and dimensions of the largest identified particles. Different pills crushers provide an homogeneous powder with particles with different dimensions. Following tests are made: Genius pills crusher: about 2,000 microns; Apex pills crusher: about 4,000 microns; Gima pills crusher: about 5,000 microns; ceramic mortar and pestle: 200-500 microns; coffee mug and metal spoon provided an irregular powder with coarse particles of about 3,000 microns. Genius, Apex and Gima pills crusher devices provide reproducible performances and an easy complete dose recovery since they are closed system. Coffee mug and metal spoon do not provide a reproducible powder and a straight forward procedure and dose loss may occur during the execution of the crushing procedure, due to tablet fragments spilled out from the cup. Ceramic mortar and pestle provides good results in terms of pulverized pancrelipase enzymes tablet particle size but a high influence of the human factor is observed.

EXAMPLE 3. Pancrelipase enzymes suspension preparation: administration vehicle

Three suspensions are prepared each with 3 pulverized tablets of pancrelipase enzymes tablet with 20,880 USP units lipase (Viokace™) in 10 mL of the following aqueous media: 1) purified (deionised) water; 2) physiological solution (0.9% NaCl); 3) tap water. All tested media properly suspend the pulverized pancrelipase enzymes tablets to generate homogeneous suspension of the particles.

EXAMPLE 4. Pancrelipase enzymes suspension preparation: delivery optimization

3 pancrelipase enzymes tablets with 16,000 USP units lipase (Viokase®) are crushed with the appropriate crushing device (see Example 2) and suspended in the volume reported in table 3 of an appropriate aqueous medium (described in Example 3 before administration through NG-tube and G-tube; as worst case, the most challenging G-tube in terms of internal diameter and length having 12 Fr as outer diameter is used (i.e. Mic Kimberly Clark Bolus). From results (reported in Table 3) it turns out that by applying a suspending time of at least 15 minutes the different types of pills crusher have no impact on the deliverability of suspended pulverized pancrelipase enzymes tablet: no clogging is observed.

This procedure allows for the maintenance of the lipase activity: activity before G-tube passage is 19.7 U USP/mg and the activity after G-tube passage is 19.8 U USP/mg; therefore complete dose recovery is assured.

The procedure is also suitable to a wide range of dose strength delivery (from 1 pancrelipase enzymes tablet with 8,000 up to 3 pancrelipase enzymes tablets with 16,000 USP lipase units). Each tablet of 8,000 U USP lipase is suspended in 2,5 mL (half teaspoon) of purified water and maintained for at least 15 minutes, further amount of water is added up to the volume reported in Table 4. It turns out that the prepared suspension is doable to NG-administration (Mic-Key) with G-tubes and NG-tubes with very small dimensions, having 5 Fr tubes (such as CORPARK), or 8 Fr (such as Tyco-Healthcare manufacturer). See Table 4.

Table 3

| # | Volume (mL) | Crusing tool | Suspending time (min) | Water | Visual inspection |
|---|---|---|---|---|---|
| 1 | 10 | Genius | 0.5 | Deionized | Ok |
| 2 | 10 | Apex | 0.5 | Deionized | Clogging |
| 3 | 10 | Gima | 5 | Deionized | Clogging |
| 4 | 10 | Mortar and pestle | 5 | Deionized | Ok |
| 5 | 10 | Mortar and pestle | 5 | Tap water | Ok |
| 6 | 10 | Mortar and pestle | 5 | Saline solution | Ok |
| 7 | 10 | Apex | 30 | Deionized | Ok |
| 8 | 10 | Gima | 30 | Deionized | Ok |
| 9 | 10 | Apex | 15 | Deionized | Ok |
| 10 | 20 | Mortar and pestle | 30 | Deionized | Ok |
| 11 | 20 | Apex | 15 | Deionized | Ok |
| 12 | 20 | Mortar and pestle | 15 | Deionized | Ok |

Table 4

| # | G-tube | NG-tube | Stoma length (cm) | Ext diam (Fr) | Strength | N° tablets | Vol (mL) | Crushing tool | Visual inspection |
|---|---|---|---|---|---|---|---|---|---|
| 1 | Mic-Key | | 0.8 | 12 | 16,000 | 3 | 20 | Apex | Ok |
| 2 | Mic-Key + Secure lok | | 0.8 | 12 | 16,000 | 3 | 20 | Apex | Ok |
| 3 | | Tyco-Healthcare | | 8 | 16,000 | 3 | 20 | Silent Knight | Ok |
| 5 | | Tyco-Healthcare | | 8 | 8,000 | 4 | 20 | Apex | Ok |
| 6 | | Tyco-Healthcare | 107 | 8 | 8,000 | 4 | 10 | Apex | Ok |
| 7 | | Corpak | 56 | 5 | 8,000 | 4 | 10 | Apex | Ok |
| 8 | | Corpak | 56 | 5 | 8,000 | | 2.5 | Apex | Ok |
| 9 | | Corpak | 56 | 5 | 8,000 | | 5 | Apex | Ok |
| 10 | | Corpak | 56 | 5 | 16,000 | 1 | 5 | Apex | Ok |
| 11 | Mic-Key + Secure lok | | 4 | 12 | 8,000 | 1 | 2.5 | Apex | Ok |
| 12 | Mic-Key + Secure lok | | 4 | 12 | 16,000 | 1 | 5 | Apex | Ok |
| 13 | Mic-Key + Secure lok | | 4 | 12 | 8,000 | 2 | 5 | Apex | Ok |
| 14 | Mic | | | 12 | 8,000 | 1 | 2.5 | Apex | Ok |
| 15 | Mic | | | 12 | 16,000 | 1 | 2.5 | Apex | Ok |
| 16 | Mic | | | 12 | 8,000 | 2 | 5 | Apex | Ok |
| 17 | | Corpak | 56 | 5 | 16,000 | 2 | 10 | Apex | Ok |
| 18 | Mic-Key + Secure lok | | 4 | 12 | 16,000 | 2 | 10 | Apex | Ok |
| 19 | Mic | | | 12 | 16,000 | 2 | 10 | Apex | Ok |
| 20 | | Corpak | 56 | 5 | 16,000 | 3 | 15 | Apex | Ok |
| 21 | Mic-Key + Secure lok | | 4 | 12 | 16,000 | 3 | 15 | Apex | Ok |
| 22 | | Corpak | 56 | 5 | 8,000 | 1 | 2.5 | Apex | Ok |

EXAMPLE 5. Pancrelipase enzymes and nutrients-(Pan+EF) composition for infusion (continuous administration)- direct addition approach

The tablet with 20,880 USP lipase units (Viokace™) is pulverized using an appropriate pills crusher device (as shown in Example 2) and directly added to feeding bag containing enteral formula Ensure® Plus. A similar Pan- EF composition is prepared with a different enteral formula: Peptamen Junior® 1.5. The approach is very simple to apply, independently by the operator and the pulverized tablets are easily transferred into the feeding bag. With this approach the pills fragments do not disperse rapidly and remained at the bottom of the feeding bag; this heterogeneous system may provide severe problems in terms of safety (unsteady dose administration), incomplete dose delivery and inhomogeneous digested nutrients delivery.

EXAMPLE 6. Pancrelipase enzymes and nutrients (Pan+ EF) composition for infusion-Pre-suspension addition approach

With this approach pancrelipase enzymes tablets (2 for EF1, 4 for EF2 and for EF3) with 10,440 U USP lipase (Viokace™) are crushed with pill crushing device, then pre-suspended in deionised water, kept for 15 minutes and then added to a package of enteral formula in the feeding bag, shaken for 15 seconds. Different enteral formulas are tested: EF3 (Ensure® Plus), EF1 (Peptamen Junior®) and EF2 (Peptamen Junior® 1.5). This approach provides an homogeneous dispersion of pancrelipase enzymes in the enteral formula, and hence the prepared composition allows a constant dose and homogenous nutrients delivery. This pancrelipase enzymes aqueous suspension is content into a infusion bag and delivery is carried out. Visual inspection of the mixture in the bag is regularly (each hour) performed and no phase separation is observed, pictures are taken. It is therefore clear that this composition is suitable to be administered via continuous infusion using feeding pump and G-tube up to at least 8 hours without markedly evident phase separation.

EXAMPLE 7. Pancrelipase enzymes and nutrients (Pan+ EF) composition for infusion-Pre-suspension addition approach

Pulverized pancrelipase enzymes is prepared with pill crushing device starting from two doses of 12,500 PhEu lipase units (Eurobiol®) and then is pre-suspended in deionised water (5.0 mL), kept for 15 minutes and then added to a package of enteral formula previously poured in the feeding bag, shaken for 15 seconds. Different enteral formulas are tested: EF3, EF1 (Peptamen Junior®) and EF2 (Peptamen Junior® 1.5). This approach provides an homogeneous dispersion of pancrelipase enzymes in the enteral formula, and hence allows a constant dose and homogenous nutrients delivery. This pan-EF composition is suitable to be administered via continuous infusion using feeding pump and G-tube up to 8-10 hours without markedly evident phase separation. Visual inspection of the mixture in the bag is regularly (each hour) performed and no phase separation is observed, pictures are taken.

EXAMPLE 8. Pancrelipase enzymes and nutrients (Pan+EF) composition for infusion-Pre-Suspension addition approach

The same approach and same conditions as in previous Example 6 are applied to prepare a suspension starting from 4 pulverized tablets of 10,440 USP lipase units, keeping it for 15 minutes, and then adding it to a package of enteral formula in the original packaging, shaking for 15 seconds and then transferring into the feeding bag. Following infant formula are tested: EF4 (Nutren® 2.0) and EF5 (TwoCal® HN): an evident phase separation is observed. This separation is not observed in the blank sample (without pancrelipase enzymes tablet addition) also tested for continuous administration: physical stability up to 16 hours is observed. Visual inspection of the mixture in the bag is regularly (each hour) performed and no phase separation is observed, pictures are taken.

EXAMPLE 9. Pancrelipase enzymes and nutrients (Pan+EF) composition for infusion-Pre-Suspension addition approach

The same approach and same conditions as in previous Example 8 are applied by preparing a pre-suspension of pulverized pancrelipase enzymes in amount of two doses of 12,500 PhEu lipase units (Eurobiol®) in purified water (10 mL), keeping it for 15 minutes, then adding it to a package of following enteral formulas previously poured into a feeding bag, shaken for 15 seconds: EF4 (Nutren® 2.0) and EF5 (TwoCal® HN). An evident phase separation is observed. This separation is not observed in the blank sample (without pancrelipase enzymes tablet addition) also tested for continuous administration: physical stability up to 16 hours is observed. Visual inspection of the mixture in the bag is regularly (each hour) performed and no phase separation is observed, pictures are taken.

EXAMPLE 10. Pancrelipase enzymes and infant formula (Pan+IF) composition for infusion- Pre-Suspension addition approach

The same approach and same conditions as in previous Example 6 are applied by adding 2 pulverized pancrelipase enzymes tablets with 10,440 USP lipase units (Viokace™) to 5 mL of purified water, keeping it for 15 minutes, and then adding the suspension to 250 mL of infant formula previously poured in the feeding bag and gently shaken for 15 seconds and then transferring into the feeding bag. Following infant formulas are tested: IF1 (Neolatte® 1), IF2 (Neolatte® 2), IF3 (Humana® 1), IF4 (Nutramigen®). An evident phase separation is observed for IF4. Whereas no separation is observed with IF1, IF2, IF3; an homogeneous stable dispersion of pancrelipase enzymes in these infant formulas is maintained for up to 8 hours; a constant dose and homogenous nutrients delivery can be accomplished with IF1, IF2, IF3 and continuous infusion using feeding pump and G-tube can be carried out. No phase separation is observed in the blank sample (without pancrelipase enzymes tablet addition) also tested for continuous administration: physical stability up to 16 hours is observed. Visual inspection of the mixture in the bag is regularly (each hour) performed and no phase separation is observed, pictures are taken. This result shows that the pancrelipase enzymes tablet can be used to prepare stable Pan+IF composition.

EXAMPLE 11. Pancrelipase enzymes and nutrients (Pan+IF) composition for infusion-Pre-Suspension addition approach

The same approach and same conditions as in previous Example 10 are applied by adding to two pulverized pancrelipase enzymes doses each of 12,500 PhEur lipase units (Eurobiol) to 5.0 mL of purified water, forming the suspension, keeping it for 15 minutes, and then added to 250 mL of infant formula already poured into the feeding bag and gently shaken for 15 seconds. Following enteral are tested: IF1 (Neolatte® 1), IF2 (Neolatte® 2), IF3 (Humana® 1), IF4 (Nutramigen®). The same results as reported in Example 10 are found here: phase separation is observed for IF4, whereas no phase separation is observed for IF1, IF2, IF3; an homogeneous stable dispersion of pancrelipase enzymes in IF1, IF2, IF3 is maintained for up to 8 hours. Visual inspection of the mixture in the bag is regularly (each hour) performed and no phase separation is observed, pictures are taken.

EXAMPLE 12. Preparation of pancrelipase enzymes suspension (Step 1)

Pancrelipase enzymes tablets with 10,440 USP lipase units (Viokace™ 10,440 USP units) are crushed one by one to generate a fine powder using a pill crushing device (Apex Ultra Pills Crusher®). The powdered pancrelipase enzymes tablets are transferred into a small glass container. ½ teaspoon (2.5 mL) of water for every tablet with 10,440 USP units of lipase dose is added. In a parallel experiment, pancrelipase enzymes tablets with 20,880 USP lipase units (Viokace™) are used: 1 teaspoon (5 mL) of water per tablet is added. The water/ pancrelipase enzymes tablet mixture is stirred with a spoon or spatula for 30 seconds to create a uniform suspension. The suspension is kept rest at room temperature for 15 minutes, in order to help the dissolution. The suspension is stirred with a spoon or spatula for a few seconds before administration. This prepared suspension is stable (lipase activity) for at least 30 minutes.

EXAMPLE 13. Administration of pancrelipase enzymes and nutrients composition by continuous infusion (Step 2)

The suspension of Example 12 is added to the feeding bag containing the prescribed amount of enteral formula/ pancrelipase enzymes tablet, the bag is shaken for 15 seconds in order to homogenize pancrelipase enzymes tablet suspension and enteral formula. The container is rinsed with an additional 10 mL of water to recover any remaining residue and administer as described above. The enteral feeding pump is inserted into the pump as per manufacturer's instructions and connected to G-tube. The pump is turned on and the correct flow rate is set. The tube is unclamped and the pump is set under operation. The feed is checked ensure that it is running and that there are no leakages from each tube connection or kink in the tube. When the feed is completed the giving set is clamped and disconnected (the pump has an alarm to indicate if there are any blockages and when the feed is completed). The prescribed water flush is administered. The extension tube is clumped and disconnected.

EXAMPLE 14. Administration of pancrelipase enzymes and nutrients (Pan+EF) composition by continuous infusion - efficiency of fluid delivery.

The cumulative volume of the delivered EF as a function of time over the feeding period with and without pancrelipase enzymes material is calculated using an appropriate graduated cylinder after G-tube delivery. Pan-EF is administered with preparation procedure described in Examples 12, 13, using a representative infusion equipment (feeding pump: Kangaroo™ ePump Enteral Feeding Pump; bag: Kangaroo Joey™ Enteral Feeding Pump Sets; G-tube: Kimberly Clark MIC-KEY 12 Fr 4.0 cm). The flow from 10 mL/h to 125 mL/h (as applied for pediatric 0-14 aged patients) is used to mimic usual clinical administration for enteral feeding. A blank administration (EF without added pancrelipase enzymes suspension) is also carried out using the same equipment. The delivered volume at given timepoints: 2, 4, 6 and 8 hours (time(h)= theoretical delivered volume (mL)/ flow pump (mL/h) is recorded. Volumes of Pan+EF mixture-composition are collected considering three different simulated administrations and compared with a blank administration at each timepoint. The efficiency of fluid delivery for Pan+EF composition at each timepoint is calculated as follows:

$$\text{Efficiency of fluid delivery (\%)} = \frac{\text{Pan+EF composition Delivered volume at time t}}{\text{blank (Enteral Formula only)Delivered volume at time t}} \times 100$$

Data in Table 5 shows that the efficiency of Pan+EF composition delivery is the same as the delivery of enteral formula alone. The % of delivered volume at time (t) *vs* blank is comprised between 95 and 105%.

Table 5

| Time (h) | Enteral formula | Flow (mL/h) | Blank (EF only) delivered volume (mL) | Pan+EF composition delivered volume (mL) | | | | | |
|---|---|---|---|---|---|---|---|---|---|
| | | | | #1 | % on blank | #2 | % on blank | #3 | % on blank |
| 2 | EF1 | 10 | 20 | 20 | 100 | 20 | 100 | 20 | 100 |
| 4 | | | 68 | 71 | 104 | 69 | 101 | 70 | 103 |
| 6 | | | 87 | 91 | 105 | 90 | 103 | 88 | 101 |
| 8 | | | 138 | 142 | 103 | 138 | 100 | 138 | 100 |
| 2 | EF1 | 125 | 252 | 260 | 103 | 245 | 97 | 255 | 101 |
| 4 | | | 520 | 535 | 103 | 510 | 98 | 530 | 102 |
| 6 | | | 770 | 800 | 104 | 760 | 99 | 782 | 102 |
| 8 | | | 1040 | 1090 | 105 | 1017 | 98 | 1040 | 100 |
| 2 | EF2 | 10 | 20 | 20 | 100 | 20 | 100 | 19 | 95 |
| 4 | | | 69 | 68 | 99 | 69 | 100 | 68 | 99 |
| 6 | | | 89 | 87 | 98 | 88 | 99 | 87 | 98 |
| 8 | | | 135 | 137 | 101 | 137 | 101 | 135 | 100 |
| 2 | EF2 | 125 | 250 | 250 | 100 | 245 | 98 | 248 | 99 |
| 4 | | | 520 | 520 | 100 | 520 | 100 | 520 | 100 |
| 6 | | | 760 | 785 | 103 | 750 | 99 | 770 | 101 |
| 8 | | | 1060 | 1048 | 99 | 1070 | 101 | 1035 | 98 |

EXAMPLE 15. Pancrelipase enzymes stability in enteral formula

The pancrelipase enzymes stability is assessed in enteral formulas over the entire feeding period (8 h) by measuring the activity of the three enzymes (lipase, protease and amylase) at given timepoints: 0, 2, 4, 8 hours. Pancrelipase enzymes suspension is mixed with the enteral formulas listed in Table 1 according to above Examples and administered using the feeding equipment as described Examples 12, 13. Administration in continuous infusion is performed using the Pan+EF composition at the low pancrelipase enzymes /EF ratio (1 tablet with 10,440 USP units lipase /250 mL EF), which represents the worst case in terms of pancrelipase enzymes stability challenge. The enzyme stability is evaluated as recovered activity at each timepoint compared with the activity found at time zero (Pan+ EF composition immediately after preparation), results are expressed as recovery percentage vs time zero.

15.1 Determination of lipase activity. Samples for lipase activity determination at 2 and 4 hours timepoints are collected both from the Pan+EF composition contained in the feeding bag and from the collected volumes delivered through G-tube and demonstrate the homogeneity of administration. Lipase activity is independent from the sampling site since it provides coherent results in terms of enzymatic stability. Samples at the endpoint are collected from the volume delivered through the G-tube.

Table 6

| Time (h)-sampling site | Enteral formula | Lipase activity USP Units/mg | | % recovery at t0 | |
|---|---|---|---|---|---|
| | | #1 10 mL/h | #2 125 mL/h | #1 | #2 |
| 0 | EF1 | 23.8 | 24.7 | NA | NA |
| 2-bag | | 22.7 | 24.9 | 95 | 101 |
| 2-tube | | 23.8 | 24.2 | 100 | 98 |
| 4-bag | | 23.1 | 23.7 | 97 | 96 |
| 4-tube | | 23.4 | 25.0 | 98 | 101 |
| 8-tube | | 23.0 | 25.0 | 97 | 101 |
| 0 | EF2 | 23.2 | 23.1 | NA | NA |
| 2-bag | | 23.4 | 23.5 | 101 | 102 |
| 2-tube | | 23.8 | 22.4 | 103 | 97 |
| 4-bag | | 24.1 | 25.5 | 104 | 110 |
| 4-tube | | 24.4 | 23.5 | 105 | 102 |
| 8-tube | | 24.2 | 24.3 | 104 | 105 |

Lipase activity remains stable in the tested formula over 8 hours. The gradual increase in the enzyme activity observed over the course of the experiment is due to an enzyme conformational change (associated with increased lipase activity) induced by the EF medium.

*15.2 Determination of protease and amylase activities* is carried out on samples of Pan+EF mixture-composition withdrawn in the bag (the composition is homogeneous during the overall administration period, see example above). Protease and amylase assays are summarized in Tables 7, 8.

Table 7

| Time (h) | Enteral formula | Protease activity USP Units/mg | | % recovery on t0 | |
|---|---|---|---|---|---|
| | | #1 10 mL/h | #2 125 mL/h | #1 | #2 |
| 0 | EF1 | 147.7 | 138 | NA | NA |
| 2 | | 142.6 | 143.6 | 97 | 104 |
| 4 | | 145.8 | 145.4 | 99 | 105 |
| 8 | | 140.6 | 137.4 | 95 | 100 |
| 0 | EF2 | 149.4 | 160.4 | NA | NA |
| 2 | | 138.8 | 149.3 | 93 | 93 |
| 4 | | 146.7 | 160.7 | 98 | 100 |
| 8 | | 139.6 | 142.4 | 93 | 89 |

Table 8

| Time (h) | Enteral formula | Amylase activity USP Units/mg | | % recovery on t0 | |
|---|---|---|---|---|---|
| | | #1 10 mL/h | #2 125 mL/h | #1 | #2 |
| 0 | EF1 | 121.9 | 123.2 | NA | NA |
| 2 | EF1 | 109.8 | 117.1 | 90 | 95 |
| 4 | EF1 | 108.0 | 110.2 | 89 | 89 |
| 8 | EF1 | 120.8 | 107.3 | 99 | 87 |
| 0 | EF2 | 136.6 | 149.6 | NA | NA |
| 2 | EF2 | 127.0 | 157.9 | 93 | 106 |
| 4 | EF2 | 130.2 | 143.5 | 95 | 96 |
| 8 | EF2 | 120.0 | 133.3 | 88 | 89 |

According to the results, lipase, protease and amylase are stable in infusion condition up to 8 hours; the activity recovery % is within 90-110 % for lipase and protease and 85-115% for amylase.

EXAMPLE 16. Digestion nutrients assessment

The digested nutrients profile in the Pan+EF compositions is determined by investigating the kinetic of digestion of nutrients induced by pancrelipase enzymes considering two aspects: 1) the decreasing of principal nutrients contained in the enteral formulas (triglycerides, total protein and maltodextrins) and 2) the increasing formation of products from the digestion of the nutrients (free fatty acids (FFA), tryptophan (AA) and short chain sugars (SCS). The change of the nutrients of the enteral formula in presence of pancrelipase enzymes is monitored and a representative marker for\ each class of digested nutrients is identified for investigating the digestion extent during the administration of the enteral formulas.

*Digestion study.* Pan+EF composition are prepared according to above Examples and administered using the feeding equipment representative of the clinical practice (Examples 12,13). Blank (EF only= no digestion) is also prepared in the same way. At given timepoints (0, 2h, 4h, 8h) both Pan+EF composition and blank are sampled from the feeding bag. Time zero is generated by sampling the suspension immediately after preparation. Digestion process is simulated for the following two different preparations: Pan+EF composition administration in continuous infusion performed using the higher pancrelipase enzymes/EF ratio, which represents the most challenging condition in terms of enteral formula modification (high enzyme activity produce a increase in digestion). The extent of digestion is described hereafter in the following paragraphs.

*16.1. Fats analysis*

16.1.1) Tryglicerides (as fat nutrients marker) amount is monitored considering triolein as marker. At each timepoint of the experiment (0, 2, 4 and 8 hours) 2 mL of each sample (EF only = blank; Pan+EF composition) is extracted to quantitatively recover the lipid fraction. Before each sampling, suspension is gently shaken for 15 seconds. The obtained results are summarized in Table 9.

Table 9

| Time (h) | Enteral formula | blank (EF only) Administration (mg of triolein in 100 mL of EF) | % respect to | Pan+EF #1 (mg of triolein in 100 mL of EF) | % respect t0 | Pan+EF #2 (mg of triolein in 100 mL of EF) | % respect t0 |
|---|---|---|---|---|---|---|---|
| 0 | EF1 | 498.1 | NA | 402.0 | NA | 405.8 | NA |
| 2 | EF1 | 503.8 | 101 | 283.6 | 71 | 297.1 | 74 |
| 4 | EF1 | 510.5 | 101 | 220.1 | 55 | 266.2 | 66 |
| 8 | EF1 | 502.4 | 98 | 127.0 | 32 | 127.1 | 32 |

(continued)

| Time (h) | Enteral formula | blank (EF only) Administration (mg of triolein in 100 mL of EF) | % respect to | Pan+EF #1 (mg of triolein in 100 mL of EF) | % respect t0 | Pan+EF #2 (mg of triolein in 100 mL of EF) | % respect t0 |
|---|---|---|---|---|---|---|---|
| 0 | EF2 | 774.3 | NA | 749.3 | NA | 785.1 | NA |
| 2 | | 786.1 | 102 | 516.6 | 69 | 564.2 | 75 |
| 4 | | 822.1 | 106 | 444.9 | 59 | 382.3 | 51 |
| 8 | | 807.7 | 104 | 200.5 | 27 | 160.4 | 21 |
| NA: Not applicable | | | | | | | |

The lipolysis activity of lipase present in the composition during the overall administration period is evident from the remarkable reduction of the triglycerides level, whereas the blank administration shows a constant triglycerides amount during the same period of time. The concentration of triolein dropped to 69-75% of the initial value after 2 hours, and it reaches about 21-32% of the initial TG (TG=triglycerides) concentration after 8 hours. After 8 hours about 30% of triolein is still present in the Pan+EF mixture-composition at the endpoint of the experiment, thus showing that hydrolysis of fatty acid is not complete; this occurs because lipolysis is inhibited by reaction products (FFA) when there is no acceptor to remove these products from the oil-water interface (micelles, bile salts, intestinal absorption), as in the tested conditions. 16.1.2) Free fatty acids (as fat digestion products marker) amount is monitored considering oleic, linoleic and palmitic acids as markers. At each timepoint of the experiment (0, 2, 4 and 8 hours) 2 mL of each sample (EF only = blank; Pan+EF composition) is extracted to quantitatively recover the lipid fraction. Before each sampling, suspension is gently shaken for 15 seconds. The obtained results are summarized in Table 10.

Table 10

| Time (h) | Enteral formula | Blank (EF only) administration (mg of oleic acid in 100 mL of EF) | Pan+EF #1 (mg of oleic acid in 100 mL of EF) | Pan+EF #2 (mg of oleic acid in 100 mL of EF) |
|---|---|---|---|---|
| 0 | EF1 | ND | 42.6 | 46.2 |
| 2 | | ND | 155.0 | 86.8 |
| 4 | | ND | 130.9 | 111.0 |
| 8 | | ND | 153.8 | 145.9 |
| 0 | EF2 | ND | 18.7 | 24.9 |
| 2 | | ND | 166.2 | 131.0 |
| 4 | | ND | 208.2 | 190.9 |
| 8 | | ND | 284.5 | 246.3 |
| ND: Not detected | | | | |

Table 11

| Time (h) | Enteral formula | Blank (EF only) administration (mg linoleic acid /100 mL EF) | Pan+EF #1 (mg linoleic acid /100 mL EF) | Pan+EF #2 (mg linoleic acid/100 mL EF) |
|---|---|---|---|---|
| 0 | EF1 | ND | 40.2 | 42.5 |
| 2 | | ND | 72.0 | 65.4 |
| 4 | | ND | 83.2 | 95.2 |
| 8 | | ND | 102.1 | 116.8 |

(continued)

| Time (h) | Enteral formula | Blank (EF only) administration (mg linoleic acid /100 mL EF) | Pan+EF #1 (mg linoleic acid /100 mL EF) | Pan+EF #2 (mg linoleic acid/100 mL EF) |
|---|---|---|---|---|
| 0 | EF2 | ND | 52.8 | 59.8 |
| 2 | | ND | 98.7 | 167.1 |
| 4 | | ND | 118.4 | 153.4 |
| 8 | | ND | 168.6 | 159.4 |
| ND. Not detected | | | | |

Table 12

| Time (h) | Enteral formula | Blank (EF only) administration (mg palmitic acid / 100 mL EF) | Pan+EF #1 (mg palmitic acid/100 mL EF) | Pan+EF #2 (mg palmitic acid /100 mL EF) |
|---|---|---|---|---|
| 0 | EF1 | ND | ND | ND |
| 2 | | ND | 36.2 | 36.9 |
| 4 | | ND | 37.5 | 48.9 |
| 8 | | ND | 43.7 | 55.8 |
| 0 | EF2 | ND | ND | 19.8 |
| 2 | | ND | 45.6 | 67.2 |
| 4 | | ND | 59.2 | 77.7 |
| 8 | | ND | 85.8 | 83.9 |
| ND: Not detected | | | | |

Enteral formulas, as per their composition, do not contain FFA as confirmed by the absence of these compounds in the HPL chromatogram of the blank; on the other side free fatty acids are detected in the chromatogram of Pan+EF mixture, confirming that lipolysis rapidly occurred already at time 0, that is immediately after Pan+EF composition was prepared.

*16.2.Proteins analysis*

16.2.1) Total proteins (as protein nutrients marker) amount is monitored using the Bradford method. At each timepoint of the experiment (0, 2, 4 and 8 hours) 2 mL of each sample (EF only = blank; Pan+EF composition) is extracted to quantitatively recover the proteic fraction. Before each sampling, suspension is gently shaken for 15 seconds. The obtained results are summarized in Table 13.

Table 13

| Time (h) | Enteral formula | Blank (EF only) administration (mg of total proteins in 1 mL of EF) | % respect t0 | Pan+EF #1 (mg of total proteins in 1 mL of EF) | % respect t0 | Pan+EF #2 (mg of total proteins in 1 mL of EF) | % respect to |
|---|---|---|---|---|---|---|---|
| 0 | EF1 | 14.1 | NA | 11.4 | NA | 12.6 | NA |
| 2 | | 13.2 | 94 | 10.6 | 93 | 11.7 | 93 |
| 4 | | 13.8 | 105 | 9.3 | 82 | 9.1 | 72 |
| 8 | | 14.4 | 104 | 7.5 | 66 | 8.0 | 63 |

(continued)

| Time (h) | Enteral formula | Blank (EF only) administration (mg of total proteins in 1 mL of EF) | % respect t0 | Pan+EF #1 (mg of total proteins in 1 mL of EF) | % respect t0 | Pan+EF #2 (mg of total proteins in 1 mL of EF) | % respect to |
|---|---|---|---|---|---|---|---|
| 0 | EF2 | 20.8 | NA | 21.5 | NA | 19.2 | NA |
| 2 | | 20.2 | 97 | 15.6 | 73 | 14.7 | 77 |
| 4 | | 19.2 | 92 | 14.4 | 67 | 13.8 | 72 |
| 8 | | 21.0 | 101 | 14.0 | 65 | 13.2 | 69 |
| NA: Not Applicable | | | | | | | |

The total protein amount remains constant during the overall administration of the blank, while a remarkable reduction of the proteins level (as effect of the proteolysis activity of protease present in pancrelipase enzymes material is observed in the Pan+EF composition: the concentration of protein drop to about 63-69% of the initial value after 8 hours. Proteolysis is not completed after 8 hours.

16.2.2) Tryptophan (as protein digestion products marker) At each timepoint of the experiment (0, 2, 4 and 8 hours) 1 mL of each sample (EF only = blank; Pan+EF composition) is extracted to quantitatively recover the amino acid fraction. Before each sampling, suspension is gently shaken for 15 seconds. The obtained results are summarized in the table 14 hereafter.

Table 14

| Time (h) | Enteral formula | Blank (EF only) administration (mg tryptophan / 100 mL EF) | Pan+EF #1 (mg tryptophan/ 100 mL EF) | Pan+EF #2 (mg tryptophan/ 100 mL EF) |
|---|---|---|---|---|
| 0 | EF1 | ND | 1.3 | 1.7 |
| 2 | | ND | 2.8 | 2.1 |
| 4 | | ND | 4.0 | 5.2 |
| 8 | | ND | 6.0 | 7.2 |
| 0 | EF2 | ND | 2.1 | 1.9 |
| 2 | | ND | 4.2 | 4.3 |
| 4 | | ND | 5.3 | 5.8 |
| 8 | | ND | 8.1 | 8.0 |
| ND: Not Detected | | | | |

Enteral formulas as per their composition do not contain tryptophan, confirmed by the absence of this aminoacid in the blank (determined by HPLC); on the other side tryptophan was detected in the Pan+EF composition (determined by HPLC) confirming that proteolysis rapidly occurred already at time 0 (immediately after Pan+EF composition is prepared).

16.3. Carbohydrates analysis

16.3.1) Maltodextrins (as carbohydrates nutrients marker) is monitored by HPLC method considering maltoheptaose (M7), maltohexaose (M6) and maltotetrahose (M4) as markers. At each timepoint of the experiment (0, 2, 4 and 8 hours) 2 mL of each sample (EF only = blank; Pan +EF mixture) is extracted to quantitatively recover the carbohydrates fraction. Before each sampling, suspension was gently shaken for 15 seconds. The results are summarized in Table 15.

Table 15

| Time (h) | Enteral formula | Blank (EF only) administration (Area % of M7) | % respect to | Pan+EF #1 (Area % of M7) | % respect to | Pan+EF #2 (Area % of M7) | % respect t0 |
|---|---|---|---|---|---|---|---|
| 0 | EF1 | 10.6 | NA | 10.0 | NA | 9.0 | NA |
| 2 | | 10.6 | 100 | 1.0 | 10 | 1.3 | 14 |
| 4 | | 9.9 | 93 | 1.4 | 14 | 2.5 | 28 |
| 8 | | 10.8 | 102 | 1.9 | 19 | 2.4 | 27 |
| 0 | EF2 | 23.8 | NA | 21.7 | NA | 227 | NA |
| 2 | | 22.5 | 95 | 2.3 | 11 | 2.8 | 12 |
| 4 | | 24.6 | 103 | 2.6 | 12 | 2.6 | 11 |
| 8 | | 26.1 | 110 | 1.8 | 8 | 1.7 | 7 |

NA: Not Applicable

Table 16

| Time (h) | Enteral formula | Blank (EF only) administration (Area % of M6) | % respect to | Pan+EF #1 (Area % of M6) | Pan+EF #2 (Area % of M6) |
|---|---|---|---|---|---|
| 0 | EF1 | 12.0 | NA | 9.0 | 10.0 |
| 2 | | 12.0 | 100 | ND | ND |
| 4 | | 11.1 | 93 | ND | ND |
| 8 | | 12.2 | 102 | ND | ND |
| 0 | EF2 | 28.4 | NA | 25.9 | 26.4 |
| 2 | | 24.0 | 85 | ND | ND |
| 4 | | 23.8 | 84 | ND | ND |
| 8 | | 25,5 | 90% | ND | ND |

NA: Not Applicable
ND: Not Detected

Table 17

| Time (h) | Enteral formula | Blank (EF only) administration (Area % of M4) | % respect to | Pan+EF #1 (Area % of M4) | Pan+EF #2 (Area % of M4) |
|---|---|---|---|---|---|
| 0 | EF1 | 3.9 | NA | 5.5 | 6.8 |
| 2 | | 4 | 103 | 0.7 | ND |
| 4 | | 5.4 | 138 | 0.7 | ND |
| 8 | | 3.7 | 95 | ND | ND |

(continued)

| Time (h) | Enteral formula | Blank (EF only) administration (Area % of M4) | % respect to | Pan+EF #1 (Area % of M4) | Pan+EF #2 (Area % of M4) |
|---|---|---|---|---|---|
| 0 | EF2 | 8.4 | NA | 9.1 | 9.0 |
| 2 | | 8.4 | 100 | ND | ND |
| 4 | | 9.2 | 110 | ND | ND |
| 8 | | 8.1 | 96 | ND | ND |
| NA: Not Applicable ND: Not Detected | | | | | |

Maltodextrins amount remained constant in the blank during the overall administration period (determined by HPLC method), while a remarkable reduction of the maltodextrins level (as effect of the amylolitic activity of amylase present in pancrelipase enzymes material) in the Panc+EF mixture is observed: the concentration of maltoheptaose rapidly dropped respect the initial value after 2 hours, additionally maltohexaose and maltotetraose resulted completely digested even only after 2 hours. Concomitantly with the decreasing of high molecular weight maltodextrins the increasing of the related short chain sugars i.e. maltose and maltotriose is observed.

16.3.2) Short chain sugars (as carbohydrates digestion products marker) amount is monitored considering maltose as marker. At each timepoint of the experiment (0, 2, 4 and 8 hours) 2 mL of each sample (EF only = blank; Pan+EF composition) is extracted to quantitatively recover the sugar fraction. Before each sampling, suspension is gently shaken for 15 seconds. The obtained results are summarized in the table hereafter.

Table 18

| Time (h) | Enteral formula | Blank (EF only) administration (mg maltose/ 100 mL EF) | Pan+EF #1 (mg maltose / 100 mL EF) | Pan+EF #2 (mg maltose/ 100 mL EF) |
|---|---|---|---|---|
| 0 | EF1 | 226.1 | 2332.2 | 2039.6 |
| 2 | | 226.8 | 2964.7 | 2907.9 |
| 4 | | 341.3 | 3324.6 | 3277.4 |
| 8 | | 191.3 | 3275.4 | 3520.7 |
| 0 | EF2 | 424.7 | 2959.5 | 2967.6 |
| 2 | | 370.3 | 4968.7 | 4854.9 |
| 4 | | 431.5 | 5181.3 | 4919.0 |
| 8 | | 402.7 | 5778.7 | 5381.3 |

Enteral formulas show a lower amount of maltose than that detected in Pan+EF composition (determined with HPLC method), confirming that amylolisis rapidly occurred already at Time 0 (immediately after Pan+EF composition is prepared). Maltose was the end-product of the attack of $\alpha$-amylase on glucose polymers. Saccharose is detected in EF1 as well since it is an ingredient of this formula, the amount of this sugar remains almost constant (see table 19) during the overall administration period, considering that saccharose is not a digestion product of amylase.

Table 19

| Time (h) | Enteral formula | Blank (EF only) administration (mg saccharose / 100 mL EF) | % respect t0 | Pan+EF #1 (mg saccharose / 100 mL EF) | % respect t0 | Pan+EF #2 (mg saccharose / 100 mL EF) | % respect t0 |
|---|---|---|---|---|---|---|---|
| 0 | EF1 | 2995.6 | NA | 2690.2 | NA | 2670.7 | NA |
| 2 | | 2927.8 | 98 | 2813.4 | 105 | 2810.7 | 104 |
| 4 | | 3256.5 | 109 | 2902.8 | 108 | 2855.2 | 106 |
| 8 | | 3004.2 | 100 | 2946.7 | 110 | 2840.0 | 106 |
| 0 | EF2 | ND | NA | ND | NA | ND | NA |
| 2 | | ND | NA | ND | NA | ND | NA |
| 4 | | ND | NA | ND | NA | ND | NA |
| 8 | | ND | NA | ND | NA | ND | NA |

NA: Not Applicable
ND: Not Detected

**Claims**

1. A process for the preparation of a stable and homogeneous liquid composition that is suitable for enteral administration comprising a digestive enzyme product which is pancrelipase and nutrients from a nutritional formula, said process comprising the following steps:

   a) preparing a suspension of digestive enzymes in aqueous solution comprising the step of:

   a.1) reducing the size of the digestive enzyme product by crushing, pulverizing or mashing;
   a.2) adding an aqueous solution;
   a.3) mixing to form the suspension; and
   a.4) keeping it for a period of time greater than 5 minutes; and

   b) mixing the suspension with a liquid nutritional formula to form the stable and homogeneous liquid composition;

   wherein the nutritional formula has a total fat and protein and carbohydrate nutrient content from 10 to 35 g /100 mL, or has a total fat and protein nutrient content from 4.5 to 11.5 g /100 mL, or has a total fat nutrient content from 3.0 to 7.0 g /100 mL, or has a total protein nutrient content from 1.3 to 6.3 g /100 mL,
   wherein the aqueous solution of step a.2) is selected from the list consisting of:

   purified water; deionized water; sterile water; tap water; and physiological saline solution,
   and wherein the aqueous solution of step a.2) is added in amount of less than 10 mL.

2. The process of claim 1, wherein the period of time of step a.4) is between 15 and 30 minutes.

3. The process of claim 1 or 2, wherein the aqueous solution of step a.2) is added in amount of about 2.5 mL for a digestive enzyme product having about 10,400 USP units of lipase, or a corresponding multiple amount of solution is added for a product having multiple USP units of lipase.

4. The process of claim 1, 2, or 3, wherein the digestive enzyme product is a non-gastroresistant product.

5. The process of claim 1, 2, 3, or 4, wherein the digestive enzyme product is either uncoated or coated.

6. The process of claim 1, 2, 3, 4, or 5, wherein the pancrelipase enzyme product is in the form of powder, granules, tablets, spheres, minitablets, microtablets, microparticles, microspheres, microcapsules or micropellets and\or wherein the pancrelipase enzyme product is an immediate release pancrelipase enzymes product or dosage form.

7. The process of claims 1, 2, 3, 4, 5, or 6, wherein the nutritional formula is adult /child formula or infant formula.

**Patentansprüche**

1. Verfahren zur Herstellung einer stabilen und homogenen flüssigen Zusammensetzung, die zur enteralen Verabreichung geeignet ist, umfassend ein Verdauungsenzymprodukt, das Pancrelipase ist, und Nährstoffe aus einer Nährstoffformel, wobei das Verfahren die folgenden Schritte umfasst:

   a) Herstellen einer Suspension von Verdauungsenzymen in wässriger Lösung, umfassend die folgenden Schritte:

      a.1) Reduzieren der Größe des Verdauungsenzymprodukts durch Zerstoßen, Pulverisieren oder Zerdrücken;
      a.2) Zusetzen einer wässrigen Lösung;
      a.3) Vermischen zur Bildung der Suspension; und
      a.4) Aufrechterhalten derselben über einen Zeitraum von über 5 min; und

   b) Vermischen der Suspension mit einer flüssigen Nährstoffformel, um die stabile und homogene flüssige Zusammensetzung zu bilden;

   wobei die Nährstoffformel einen Fett- und Protein- und Kohlenhydrat-Nährstoffgesamtgehalt von 10 bis 35 g/100 ml aufweist oder einen Fett- und Protein-Nährstoffgesamtgehalt von 4,5 bis 11,5 g/100 ml aufweist oder einen Fettnährstoffgesamtgehalt von 3,0 bis 7,0 g/100 ml oder einen Fettnährstoffgesamtgehalt von 1,3 bis 6,3 g/100 ml aufweist,
   wobei die wässrige Lösung aus Schritt a.2) in einer Menge von weniger als etwa 10 ml zugesetzt wird.

2. Verfahren nach Anspruch 1, wobei der Zeitraum für Schritt a.2) zwischen 15 und 30 min beträgt.

3. Verfahren nach Anspruch 1 oder 2, wobei die wässrige Lösung aus Schritt a.2) in einer Menge von etwa 2,5 ml für ein Verdauungsenzymprodukt mit etwa 10.400 USP-Lipase-Einheiten zugesetzt wird oder ein entsprechendes Vielfaches der Menge der Lösung für ein Produkt mit einem Vielfachen an USP-Lipase-Einheiten zugesetzt wird.

4. Verfahren nach Anspruch 1, 2 oder 3, wobei das Verdauungsenzymprodukt ein verdauungssaftresistentes Produkt ist.

5. Verfahren nach Anspruch 1, 2, 3 oder 4, wobei das Verdauungsenzymprodukt entweder unbeschichtet oder beschichtet ist.

6. Verfahren nach Anspruch 1, 2, 3, 4 oder 5, wobei das Pancrelipase-Enzymprodukt in Form von Pulver, Granulat, Tabletten, Kügelchen, Minitabletten, Mikrotabletten, Mikropartikeln, Mikrokügelchen, Mikrokapseln oder Mikropellets vorliegt und/oder wobei das Pancrelipase-Enzymprodukt ein Pancrelipase-Enzymprodukt oder eine solche Dosierform mit unmittelbarer Freisetzung ist.

7. Verfahren nach Anspruch 1, 2, 3, 4, 5 oder 6, wobei die Nährstoffformel eine Formel für Erwachsene/Kinder oder eine Formel für Kleinkinder ist.

**Revendications**

1. Procédé de préparation d'une composition liquide stable et homogène qui est adaptée pour une administration entérale comprenant un produit enzymatique digestif qui est la pancrélipase et des nutriments à partir d'une formule nutritionnelle, ledit procédé comprenant les étapes suivantes consistant à :

   a) préparer une suspension d'enzymes digestives dans une solution aqueuse, comprenant les étapes consistant à :

EP 2 996 712 B1

a.1) réduire la taille du produit enzymatique digestif en l'écrasant, en le pulvérisant ou en le broyant ;
a.2) ajouter une solution aqueuse ;
a.3) mélanger pour former la suspension ; et
a.4) la maintenir pendant une période de temps supérieure à 5 minutes ; et

b) mélanger la suspension avec une formule nutritionnelle liquide pour former la composition liquide stable et homogène ;

dans lequel la formule nutritionnelle a une teneur nutritives totale en matières grasses, en protéines et hydrates de carbone de 10 à 35 g/100 mL, ou a une teneur nutritive totale en matières grasses et en protéines de 4,5 à 11,5 g/100 mL ou une teneur nutritive en matières grasses totale de 3,0 à 7,0 g/100 mL, ou a une teneur nutritive totale en protéines de 1,3 à 6,3 g/100 mL,
dans lequel la solution aqueuse de l'étape a.2) est choisie dans la liste comprenant : de l'eau purifiée ; de de l'eau déminéralisée ; de l'eau stérile ; de l'eau du robinet ; et une solution saline physiologique,
et dans lequel la solution aqueuse de l'étape a.2) est ajoutée en une quantité inférieure à 10 ml.

2. Procédé selon la revendication 1, dans lequel la période de temps de l'étape a.4) est comprise entre 15 et 30 minutes.

3. Procédé selon la revendication 1 ou 2, dans lequel la solution aqueuse de l'étape a.2) est ajoutée en une quantité d'environ 2,5 ml pour un produit enzymatique digestif ayant environ 10 400 unités USP de lipase, ou une quantité multiple correspondante de solution est ajoutée pour un produit ayant plusieurs unités USP de lipase.

4. Procédé selon la revendication 1, 2 ou 3, dans lequel le produit enzymatique digestif est un produit non gastro-résistant.

5. Procédé selon la revendication 1, 2, 3 ou 4, dans lequel le produit enzymatique digestif est non enrobé ou enrobé.

6. Procédé selon la revendication 1, 2, 3, 4 ou 5, dans lequel le produit enzymatique de pancrélipase est sous la forme de poudre, de granules, de comprimés, de sphères, de mini-comprimés, de micro-comprimés, de micro-particules, de micro-sphères, de micro-capsules ou de micro-pastilles et/ou dans lequel le produit enzymatique de pancrélipase est un produit enzymatique de pancrélipase à libération immédiate ou sous une forme posologique.

7. Procédé selon les revendications 1, 2, 3, 4, 5 ou 6, dans lequel la formule nutritionnelle est une formule pour adulte/enfant ou une formule pour nourrisson.

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6051220 A
- US 20040057944 A
- US 20010046493 A
- WO 2006044529 A
- WO 2012042372 A
- WO 2012019186 A
- WO 0170047 A

**Non-patent literature cited in the description**

- **FERRIE et al.** *Nutr. Clin. Pract.,* 2011, vol. 26 (3), 349-51